# EUROPÄISCHE PATENTSCHRIFT

(11) **EP 0 808 294 B1**
(45) Veröffentlichungstag und Bekanntmachung des Hinweises auf die Patenterteilung: **30.12.1998**
(21) Anmeldenummer: 96902933.9
(22) Anmeldetag: 29.01.1996
(51) Int. Cl.: C01G 51/04, H01M 4/52

(54) **KOBALTMETALL-HALTIGES KOBALT(II)-OXID, VERFAHREN ZU SEINER HERSTELLUNG SOWIE DESSEN VERWENDUNG**
COBALTOUS OXIDE CONTAINING METALLIC COBALT, METHODS FOR PRODUCING THE SAME AND USE THEREOF
OXYDE COBALTEUX CONTENANT DU COBALT METALLIQUE, SES PROCEDES DE PRODUCTION ET SON UTILISATION

(30) Priorität: 10.02.1995 DE 19504320
(43) Veröffentlichungstag der Anmeldung: 26.11.1997
(73) Patentinhaber: H.C. Starck GmbH & Co. KG, 38642 Goslar (DE)
(72) Erfinder: GÖRGE, Astrid, D-38640 Goslar (DE); PLAGA, Katrin, D-38640 Goslar (DE); OLBRICH, Armin, D-38723 Seesen (DE)
(74) Vertreter: Steiling, Lothar, Dr.
(86) Internationale Anmeldenummer: EP9600337
(87) Internationale Veröffentlichungsnummer: WO9624557

(56) Entgegenhaltungen:
- EP-A- 0 638 520
- DE-A- 2 327 180
- US-A- 3 775 352
- CHEMICAL ABSTRACTS, vol. 96, no. 14, 5.April 1982 Columbus, Ohio, US; abstract no. 114829, KHOKHLACHEVA: "production and thermal decomposition of formates of some noferrous metals " XP002002765 & DEPOSITED DOC. VINITI, 1980, Seiten 4960-4980,

## Beschreibung

Die vorliegende Erfindung betrifft metallisches Kobalt enthaltendes Kobalt(II)-Oxid, Verfahren zu seiner Herstellung sowie dessen Verwendung.

Kobalt(II)-Oxid wird in Mischung mit metallischem Kobalt als Additiv in der positiven Masse von wiederaufladbaren alkalischen Ni-Batterien auf Basis Ni/Cd oder Ni/NiH eingesetzt. Hierzu wird Ni(OH)₂ mit der Co(II)-Oxid-Metallmischung und Hilfsstoffen zu Pasten verarbeitet, welche anschließend in einen elektrisch leitfähigen Elektrodenträger eingearbeitet werden. Die auf diesem Wege hergestellten Elektroden werden durch Trocknen und/oder Sintern weiterverarbeitet, um somit zu Batterien verschiedener Ausführung zu gelangen.

So werden beispielsweise für die Herstellung von Knopfzellen die elektrochemisch aktiven Elektrodenbestandteile zusammen mit Hilfsstoffen, vornehmlich Graphit oder Nickelpulver, zu Tabletten unterschiedlicher Größe verpreßt. Die Anteile des Kobalts in den Elektrodenmassen liegen dabei zwischen 2 bis 10 Gew.-%.

Die Hauptwirkung des Kobaltmetalls beruht laut der EP-A 353 837 darauf, daß während der ersten Ladezyklen (Formierzyklen) das Kobaltmetall entsprechend seines Potentials zunächst zu zweiwertigem Kobalt oxidiert wird und sich dadurch im alkalischen Elektrolyten lösen kann. Die so erhaltenen und eventuell bereits vorliegenden Co²⁺-Ionen diffundieren dann in Richtung der Oberfläche des Nickelhydroxids. Hier werden sie bei weiterer Ladung der Batterie zu Co³⁺-Ionen in Form von CoO(OH) oxidiert. Dieses wiederum bildet sich als Schicht auf der Oberfläche der Nickelhydroxid-Partikel aus und bewirkt bei den folgenden Ladeund Entladezyklen die elektrische Leitfähigkeit des Elektrodenmaterials.

Co²⁺-Ionen können aber ebenso in das Schichtgitter des Nickelhydroxids gelangen und dort die Eigenschaften des Hydroxids so modifizieren, daß eine höhere Ladeeffizienz des Elektrodenmaterials bewirkt wird. Zusätzlich zu den bereits genannten Eigenschaften kann das in der Elektrodenmasse eingesetzte Kobalt als Sicherheitsreserve bei zu starker Entladung wirken. Hierbei werden Co²⁺-Ionen elektrochemisch wieder reduziert und verhindern somit eine Wasserstoffentwicklung. Kobaltverbindungen mit den oben erwähnten Eigenschaften werden ebenfalls in den Patentschriften US-A 5 032 475 sowie US-A 5 053 292 und der europäischen Patentanmeldung EP-A 523 284 offenbart.

Für die Lade- und Entladezyklen bei der elektrochemischen Oxidation wird das Kobaltmetallpulver nur bis zu ca. 50 % in der Elektrode nutzbar gemacht, da der überwiegende Teil des Kobalts mit einer stabilen Oxidschicht überzogen ist. Diese Schutzschicht wiederum verhindert die Bildung von Co²⁺-Ionen, die wie bereits erwähnt für die Aktivierung der Elektrode notwendig ist. Um diese Schwierigkeit zu umgehen, wurden bislang lösliche Kobaltverbindungen wie Kobalthydroxid oder -monoxid in die Elektrodenmasse mit eingearbeitet. Dies bewirkte, daß schon vor der elektrochemischen Formierung Co²⁺-Ionen im Elektrolyten gelöst vorliegen und diese sich an der Oberfläche des Nickelhydroxids bereits abscheiden können (Matsumo et al.: The 162nd ECS Fall Meeting Detroit, 18 (1982)).

Nach diesem Stand der Technik wird das für die oben beschriebenen Anwendungszwecke verwendete Co(II)-Oxid technisch durch die thermische Zersetzung von Kobaltcarbonat, Cobalthydroxid oder höheren Cobaltoxiden hergestellt. Diese enthalten jedoch - dem thermodynamischen Gleichgewicht folgend - stets einen Überschuß an Sauerstoff und somit Restgehalte von Co(III).

Geringe Spuren von Co(III) im Co(II)-Oxid katalysieren die Oxidation des zweiwertigen zu dreiwertigem Kobalt. Dieses bildet jedoch keine im Elektrolyten löslichen Verbindungen, so daß eine Ausbildung der leitfähigen Schicht nach dem oben beschriebenen Mechanismus nicht möglich ist. Hieraus folgt, daß eine hohe Nutzbarkeit der Elektrode nur dann erzielt werden kann, wenn die Co(III)-Anteile des Ausgangsmaterials so gering wie möglich sind.

Aufgabe der vorliegenden Erfindung ist es daher, ein Kobaltmetall-haltiges Co(II)-Oxid bereitzustellen, welches die beschriebenen Nachteile nicht aufweist.

Entsprechende Co(II)-Oxide konnten nun erhalten werden durch ein Verfahren zur Herstellung von metallisches Kobalt enthaltendem Co(II)-Oxid, wobei kristallisierte basische Kobalt-Verbindungen der allgemeinen Formel

Co[(OH)₂]ₐ [O]_{b} [CO₃]_{c},

wobei die Summe von a+b+c ≥ 1 ≤1,5 beträgt,
mit wäßrigen und/oder alkoholischen Lösungen einer mindestens eine Carboxylgruppe enthaltenden organischen Verbindung umgesetzt und die dabei enthaltenen Feststoffe aus der Suspension abgetrennt und calciniert werden. Ein solches Verfahren ist Gegenstand der vorliegenden Erfindung.

Bevorzugt können beim erfindungsgemäßen Verfahren als mindestens eine Carboxylgruppe-enthaltende organische Verbindung Carbonsäuren eingesetzt werden. Dabei sind aus der Gruppe der Carbonsäuren insbesondere
- lineare oder verzweigte, gesättigte oder ungesättigte Monocarbonsäuren mit einer Anzahl der C-Atome von 1 bis 9 und/oder
- lineare oder verzweigte, gesättigte oder ungesättigte Polycarbonsäuren mit einer Anzahl der C-Atome von 2 bis 10 und/oder
- cyclische oder heterocyclische, gesättigte oder ungesättigte Mono- und Polycarbonsäuren mit einer Anzahl der C-Atome von 4 bis 14 und/oder
- lineare oder verzweigte, gesättigte oder ungesättigte Mono- und Polyhydroxycarbonsäuren mit einer Anzahl der C-Atome von 2 bis 7 und/oder
- aromatische Hydroxycarbonsäuren mit einer Anzahl der C-Atome von 7 bis 11 und/oder
- cyclische oder aliphatische, gesättigte oder ungesättigte Ketocarbonsäuren mit einer Anzahl der C-Atome von 2 bis 14 geeignet.

Ebenso vorteilhaft können Adipinsäuren, Bernsteinsäure, Glutarsäure, Glyoxylsäure, Maleinsäure, Malonsäure, Milchsäure, Oxalsäure, Phthalsäuren, Schleimsäure, Sorbinsäure, Traubensäure, Versaticsäure, Weinsäure und/oder Zitronensäure eingesetzt werden.

In einer weiteren vorteilhaften Ausführungsform des erfindungsgemäßen Verfahrens können auch die Carbonsäuren in teilveresterter Form eingesetzt werden, solange sie noch mindestens eine aktive Carboxylgruppe aufweisen.

Kristallisierte basische Kobalt-Verbindungen im Sinne dieser Erfindung weisen die Formel

Co[(OH)₂]ₐ[O]_{b}[CO₃]_{C}

auf, wobei die Summe von a + b + c ≥ 1 ≤ 1,5 beträgt. Die Werte für a, b und c können jeweils beliebig zwischen 0 und 1,5 liegen. Sie können somit sowohl als reine als auch als Mischkristalle eingesetzt werden, wobei das Kobalt in den Oxidationsstufen zwischen 2 und 3 vorliegen kann. Es eignen sich insbesondere solche mit Teilchengrößen im Bereich von 0,5 µm, vorzugsweise 2 bis 20 µm. Besonders bevorzugt liegen sie in enger Kornverteilung vor.

Die äußere Form der kristallisierten basischen Kobalt-Verbindungen bestimmen auch die Form der erfindungsgemäßen Endprodukte. Besonders bevorzugt werden solche mit sphärischer Morphologie eingesetzt.

Die das Verfahren abschließende Calcination wird vorteilhaft in Inertgasatmosphäre bei Temperaturen zwischen 200 und 1000°C, bevorzugt 500 bis 800°C, durchgeführt.

Je nach Prozeßführung erlaubt das erfindungsgemäße Verfahren sowohl eine selektive Umwandlung der äußeren Oberfläche der Teilchen als auch zusätzlich der inneren Oberfläche.

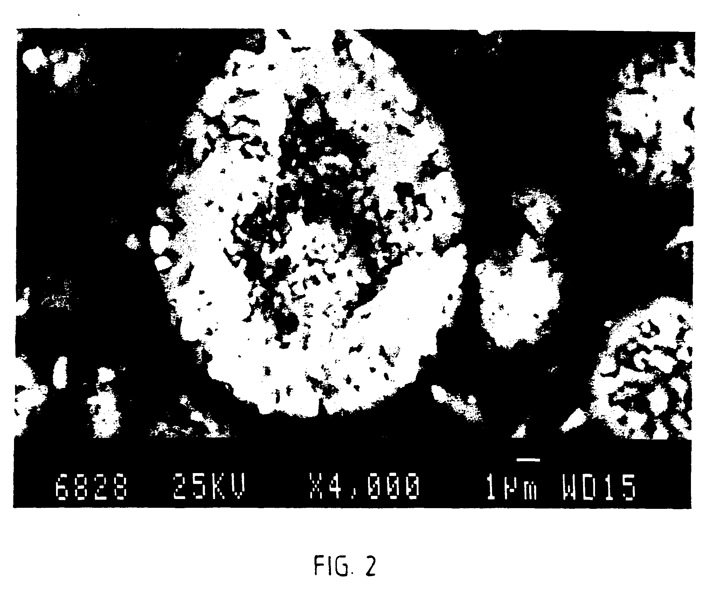

Um Co(II)-Oxid zu erhalten, wobei das metallische Kobalt sich überwiegend auf der äußeren Oberfläche des Co(II)-Oxides befindet, wird die erfindungsmäßige Umsetzung in einem Temperaturbereich von 50 bis 100°C, vorzugsweise 70 bis 90°C vorgenommen. Gegenstand dieser Erfindung sind entsprechend erhältliche Co(II)-Oxide. Eine rasterelektronenmikroskopische Aufnahme von Schliffen entsprechender Co(II)-Oxide ist in Fig. 2 dargestellt. Typisch für sie sind diskret in der äußeren Oberfläche angereicherte metallische Kobalt-Partikel im Submicron-Bereich.

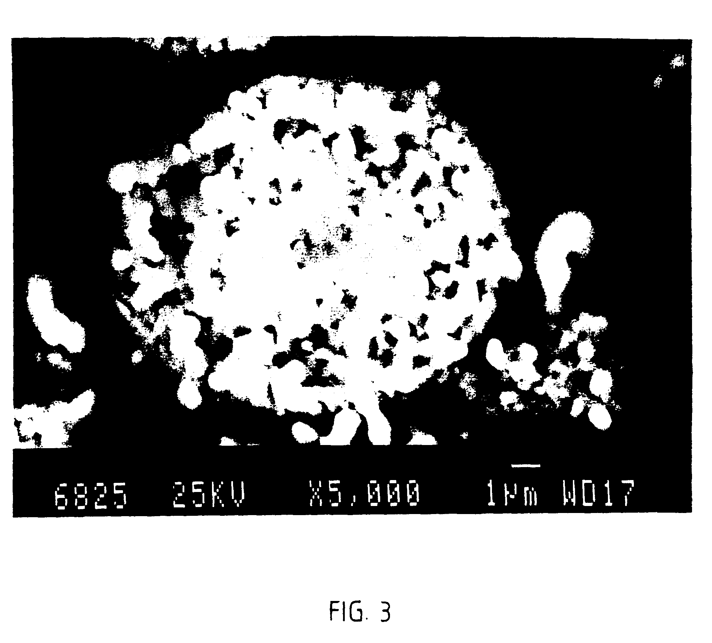

Gegenstand dieser Erfindung sind auch Co(II)-Oxide, in denen das metallische Kobalt sowohl in der inneren als auch in der äußeren Oberfläche angereichert vorliegt. Ein entsprechendes Co(II)-Oxid ist ebenso als Schliffaufnahme in Fig. 3 dargestellt. Es ist erhältlich gemäß dem erfindungsgemäßen Verfahren, wobei die Umsetzung zunächst 0,1 h bis 3 h, vorzugsweise 0,5 h bis 1,5 h, bei Raumtemperatur und anschließend 0,1 h bis 3 h bei 50 bis 100°C, vorzugsweise 70 bis 90°C, vorgenommen wird. Typisch für diese Co(II)-Oxide ist ein dreidimensionales Netzwerk aus feinverteiltem, submicronen metallischen Kobalt.

Durch das Verhältnis der entsprechenden Reduktionsäquivalente läßt sich der Anteil des metallischen Kobalts im erfindungsgemäßen Co(II)-Oxid in beliebigen Verhältnissen einstellen. Bevorzugt sind Gehalte an metallischem Kobalt von 2 bis 50 Gew.-%, bevorzugt 3 bis 20 Gew.-%.

Die erfindungsgemäßen Co(II)-Oxide zeichnen sich durch hohe Beständigkeit gegen Luftsauerstoff aus. Weitere Vorzüge des Materials liegen beispielsweise bei kugelförmigen Teilchen in ihrer hohen Fließfähigkeit, da während des gesamten Reaktionsverlaufes der Habitus der Materialien erhalten bleibt.

Desweiteren ist ein Vorteil darin zu sehen, daß die metallischen und oxidischen Anteile - im Vergleich zu den nach dem Stand der Technik produzierten Materialien - nicht entmischbar sind, und somit eine gleichmäßige Verarbeitung des Kobalts im für die Darstellung der Elektroden notwendigen Pastiervorgang ermöglicht wird.

Gegenstand dieser Erfindung ist auch die Verwendung der erfindungsgemäßen Co(II)-Oxide als Elektrodenmaterial in elektrochemischen Sekundärzellen.

Im folgenden wird die Erfindung beispielhaft erläutert, ohne daß hierin eine Einschränkung zu sehen ist.

### Beispiel 1 (Außenbeschichtung)

200 g sphärisches basisches Kobaltcarbonat, hergestellt durch Umsetzung von CoCl₂ mit Nₐ₂CO₃, wurden in 1,5 1 Wasser aufgeschlämmt und diese Suspension unter Rühren auf 85°C erhitzt. Zu dieser Suspension wurden innerhalb von 30 Minuten 100 g feste Weinsäure gleichmäßig zudosiert, wobei die Temperatur von 85°C beibehalten wurde. Anschließend wurde die Reaktionsmischung bei T = 85°C 15 Minuten gerührt und dann heiß filtriert. Der Filterkuchen wurde mit 500 ml Wasser nachgewaschen und bei T = 70°C bis zur Gewichtskonstanz getrocknet.

Es wurden 260 g basisches Kobalttartrat/carbonat als Zwischenprodukt erhalten.

100 g dieses Produktes wurden im Quarzschiffchen bei T = 700°C 3 h unter Argon calciniert.

Als Calcinationsprodukt entstanden 67 g Kobaltmetall-haltiges Co(II)-Oxid mit einem Kobaltgehalt von 79,72 %.

### Beispiel 2 (Außenbeschichtung)

200 g sphärisches basisches Kobaltcarbonat, hergestellt wie in Beispiel 1 beschrieben, wurden in 1000 ml Methanol aufgeschlämmt. Hierzu wurden 20 g Zitronensäuremonohydrat - gelöst in 50 ml Methanol - unter Rühren zugesetzt. Die Suspension wurde anschließend 4 h unter Rückfluß erhitzt, filtriert und mit 1 000 ml Methanol gewaschen. Der Filterkuchen wurde bei T = 70°C bis zur Gewichtskonstanz getrocknet.

Es ergaben sich 212 g basisches Kobaltcitrat/carbonat als Zwischenprodukt, welches bei 700°C 2 h unter Argonatmosphäre calciniert wurde. Das erhaltene Kobaltmetall-haltige Co(II)-Oxid (27 g ) weist einen Kobaltgehalt von 79,3 % auf.

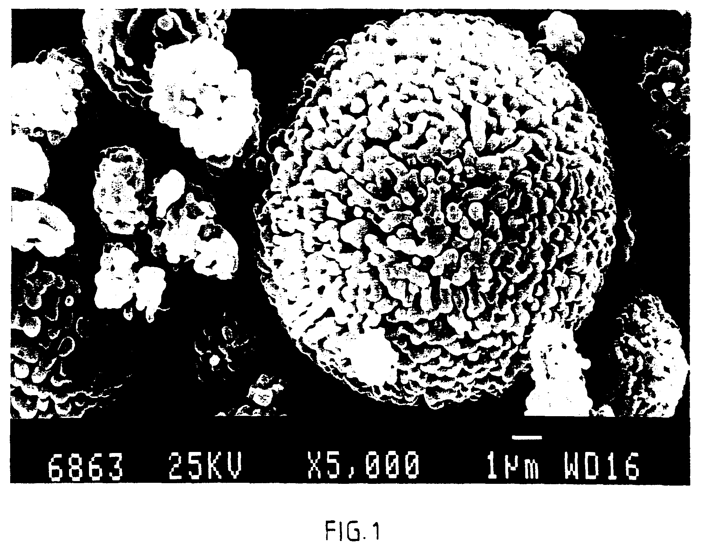

Ein entsprechend hergestelltes Pulver ist in Fig. 1 (5000-fache Vergrößerung) dargestellt.

### Beispiele 3 bis 6 (Außenbeschichtung)

200 g sphärisches basisches Kobaltcarbonat, hergestellt wie in Beispiel 1 beschrieben, wurden in Wasser suspendiert und auf T = 80°C erhitzt. Anschließend erfolgte der Zusatz der in Tabelle 1 angegebenen Menge Oxalsäure. Nach erfolgter Reaktion wurde die Suspension 1 h gerührt, filtriert und bei T = 70°C bis zur Gewichtskonstanz getrocknet.

Die auf diesem Wege erhaltenen Materialien wurden anschließend bei einer Temperatur von 700°C der Calcination unter Inertgas unterworfen.

**Tabelle 1**

| Ausgangsmaterial: sphärisches Co-Carbonat Co-Gehalt: 54,4 % Einwaage: 200 g | | |
|---|---|---|
| Beispiel Nr. | Einwaage an Oxalsäure | Kobaltgehalt im Endprodukt |
| 3 | 46 g | 81,5 % |
| 4 | 70 g | 83,1 % |
| 5 | 81 g | 83,8 % |
| 6 | 93 g | 85,0 % |

Ein entsprechend Beispiel 5 hergestelltes Pulver ist in Form eines Schliffes Fig. 2 (4000-fache Vergrößerung) dargestellt.

### Beispiel 7 (Außen- und Innenbeschichtung)

200 g sphärisches basisches Kobaltcarbonat, hergestellt wie in Beispiel 1 beschrieben, wurden in 1,5 l Wasser aufgeschlämmt und mit 100 g Weinsäure versetzt. Diese Suspension wurde 1 h bei Raumtemperatur gerührt und anschließend innerhalb von 2,5 h bis auf 53°C erhitzt. Anschließend wurde filtriert und mit 500 ml Wasser nachgewaschen. Der Filterrückstand ergab nach dem Trocknen bis zur Gewichtskonstanz 261 g basisches Kobalttartrat/carbonat als Zwischenprodukt.

Dieses wurde bei T = 700°C unter Argonatmosphäre 3 h calciniert. Als Calcinationsprodukt entstanden 137 g Kobaltmetall-haltiges Co(II)-Oxid, mit einem Kobaltgehalt von 91,4 %.

### Beispiel 8 (Außen- und Innenbeschichtung)

200 g sphärisches basisches Kobalt(II, III)-hydroxid-oxid mit einem Co-Gehalt von 49 Gew.-% Kobalt wurden mit 1,5 l Wasser aufgeschlämmt und mit 15 g fester Weinsäure versetzt. Die entstandene Suspension wurde 1,5 h bei Raumtemperatur gerührt und anschließend innerhalb von 2 h auf 70°C erhitzt. Danach wurde filtriert, gewaschen und bis zur Gewichtskonstanz getrocknet. Die aus der Reaktion erhaltenen 212 g basisches Kobalttartrat/hydroxid-oxid(II, III) wurden 3 h der Calcination bei T = 700°C unter Inertgas unterworfen.

Als Produkt wurden 117 g Kobalt-haltiges Co(II)-Oxid mit einem Kobaltgehalt von 80,8 % hergestellt.

Ein dementsprechend hergestelltes Pulver ist in Form eines Schliffes in Fig. 3 (5000-fache Vergrößerung) dargestellt.

## Patentansprüche

1. Verfahren zur Herstellung von metallisches Kobalt enthaltendem Co(II)-Oxid, dadurch gekennzeichnet, daß kristallisierte basische Kobalt-Verbindungen der allgemeinen Formel
Co[(OH)₂]ₐ [O]_{b} [CO₃]_{c},
wobei
die Summe von a+b+c ≥ 1 ≤ 1,5 beträgt,
mit wäßrigen und/oder alkoholischen Lösungen einer mindestens eine Carboxylgruppe enthaltenden organischen Verbindung umgesetzt wird und die dabei enthaltenen Feststoffe aus der Suspension abgetrennt und calciniert werden.

2. Verfahren gemäß Anspruch 1, dadurch gekennzeichnet, daß als mindestens eine Carboxylgruppe-enthaltende organische Verbindung Carbonsäuren eingesetzt werden.

3. Verfahren gemäß Anspruch 2, dadurch gekennzeichnet, daß als Carbonsäuren
- lineare oder verzweigte, gesättigte oder ungesättigte Monocarbonsäuren mit einer Anzahl der C-Atome von 1 bis 9 und/oder
- lineare oder verzweigte, gesättigte oder ungesättigte Polycarbonsäuren mit einer Anzahl der C-Atome von 2 bis 10 und/oder
- cyclische oder heterocyclische, gesättigte oder ungesättigte Monound Polycarbonsäuren mit einer Anzahl der C-Atome von 4 bis 14 und/oder
- lineare oder verzweigte, gesättigte oder ungesättigte Mono- und Polyhydroxycarbonsäuren mit einer Anzahl der C-Atome von 2 bis 7 und/oder
- aromatische Hydroxycarbonsäuren mit einer Anzahl der C-Atome von 7 bis 11 und/oder
- cyclische oder aliphatische, gesättigte oder ungesättigte Ketocarbonsäuren mit einer Anzahl der C-Atome von 2 bis 14
eingesetzt werden.

4. Verfahren gemäß einem der Ansprüche 2 oder 3, dadurch gekennzeichnet, daß als Carbonsäuren vorzugsweise Adipinsäure, Bernsteinsäure, Glutarsäure, Glyoxylsäure, Maleinsäure, Malonsäure, Milchsäure, Oxalsäure, Phthalsäuren, Schleimsäure, Sorbinsäure, Traubensäure, Versaticsäure, Weinsäure und/oder Zitronensäure eingesetzt werden.

5. Verfahren gemäß einem oder mehreren der Ansprüche 1 bis 4, dadurch gekennzeichnet, daß als mindestens eine Carboxylgruppe enthaltende organische Verbindung teilveresterte Carbonsäuren gemäß den Ansprüchen 2 bis 4 eingesetzt werden.

6. Verfahren gemäß einem oder mehreren der Ansprüche 1 bis 5, dadurch gekennzeichnet, daß die kristallisierten basischen Co-Verbindungen Teilchengrößen im Bereich von 0,5 µm bis 50 µm, vorzugsweise 2 bis 20 µm, aufweisen.

7. Verfahren gemäß einem oder mehreren der Ansprüche 1 bis 6, dadurch gekennzeichnet, daß als kristallisierte basische Co-Verbindungen solche mit sphärischer Morphologie eingesetzt werden.

8. Verfahren gemäß einem oder mehreren der Ansprüche 1 bis 7, dadurch gekennzeichnet, daß die Calcination in Inertgasatmosphäre bei Temperaturen zwischen 200 bis 1000°C, vorzugsweise zwischen 500 bis 800°C, durchgeführt wird.

9. Verfahren gemäß einem oder mehreren der Ansprüche 1 bis 8, dadurch gekennzeichnet, daß die Umsetzung in einem Temperaturbereich von 50 bis 100°C, vorzugsweise 70 bis 90°C, vorgenommen wird, wobei bei der anschließenden Calcination das metallische Kobalt überwiegend auf der äußeren Oberfläche des Co(II)-Oxides gebildet wird.

10. Verfahren gemäß einem oder mehreren der Ansprüche 1 bis 8, dadurch gekennzeichnet, daß die Umsetzung zunächst 0,1 h bis 3 h, vorzugsweise 0,5 bis 1,5 h, bei Raumtemperatur und anschließend 0,1 h bis 3 h bei 50 bis 100°C, vorzugsweise bei 70 bis 90°C, vorgenommen wird, wobei nach der anschließenden Calcination sowohl die innere, als auch die äußere Oberfläche des Co(II)-Oxides mit dem metallischen Kobalt belegt ist.

11. Metallisches Kobalt enthaltendes Co(II)-Oxid, wobei das metallische Kobalt überwiegend auf der äußeren Oberfläche des Co(II)-Oxides vorliegt, erhältlich gemäß einem oder mehreren der Ansprüche 1 bis 9.

12. Metallisches Kobalt enthaltendes Co(II)-Oxid, wobei das metallische Kobalt sowohl auf der äußeren, als auch auf der inneren Oberfläche des Co(II)-Oxides vorliegt, erhältlich gemäß einem oder mehreren der Ansprüche 1 bis 9.

13. Verwendung des metallischen Kobalt enthaltendem Co(II)-Oxides, gemäß der Ansprüche 1 bis 8 und 10, als Elektrodenmaterial in Sekundärzellen

## Claims

1. A process for producing Co(II) oxide containing metallic Co, characterised in that crystallised basic cobalt compounds of general formula
Co[(OH)₂]ₐ [O]_{b} [CO₃]_{C},
wherein
the sum of a+b+c is ≥ 1 ≤ 1.5,
are reacted with aqueous and/or alcoholic solutions of an organic compound containing at least one carboxyl group and the solids thus obtained are separated from the suspension and calcined.

2. A process according to claim 1, characterised in that carboxylic acids are used as the organic compound containing at least one carboxyl group.

3. A process according to claim 2, characterised in that
- linear or branched, saturated or unsaturated monocarboxylic acids having a number of C atoms from 1 to 9, and/or
- linear or branched, saturated or unsaturated polycarboxylic acids having a number of C atoms from 2 to 10, and/or
- cyclic or heterocyclic, saturated or unsaturated mono- and polycarboxylic acids having a number of C atoms from 4 to 14, and/or
- linear and branched, saturated or unsaturated mono- and polyhydroxycarboxylic acids having a number of C atoms from 2 to 7, and/or
- aromatic hydroxycarboxylic acids having a number of C atoms from 7 to 11, and/or
- cyclic or aliphatic, saturated or unsaturated ketocarboxylic acids having a number of C atoms from 2 to 14
are used as the carboxylic acids.

4. A process according to either one of claims 2 or 3, characterised in that adipic acid, succinic acid, glutaric acid, glyoxylic acid, maleic acid, malonic acid, lactic acid, oxalic acid, phthalic acids, mucic acid, sorbic acid, racemic acid, versatic acid, tartaric acid and/or citric acid are preferably used as the carboxylic acids.

5. A process according to one or more of claims 1 to 4, characterised in that partially esterified carboxylic acids according to claims 2 to 4 are used as the organic compound containing at least one carboxyl group.

6. A process according to one or more of claims 1 to 5, characterised in that the crystallised basic Co compounds have particle sizes within the range of 0.5 µm to 50 µm, preferably 2 to 20 µm.

7. A process according to one or more of claims 1 to 6, characterised in that compounds having spherical morphology are used as the crystallised basic Co compounds.

8. A process according to one or more of claims 1 to 7, characterised in that calcination is effected in an inert gas atmosphere at temperatures between 200 and 1000°C, preferably between 500 and 800°C.

9. A process according to one or more of claims 1 to 8, characterised in that the reaction is conducted within a temperature range of 50 to 100°C, preferably 70 to 90°C, wherein on subsequent calcination metallic cobalt is predominantly formed on the external surface of the Co(II) oxide.

10. A process according to one or more of claims 1 to 8, characterised in that the reaction is first conducted for 0.1 to 3 hours, preferably 0.5 to 1.5 hours, at room temperature, and subsequently for 0. to 3 hours at 50 to 100°C, preferably at 70 to 90°C, wherein after subsequent calcination both the internal and the external surface of the Co(II) oxide is covered with metallic cobalt.

11. Co(II) oxide containing metallic cobalt, wherein the metallic cobalt is predominantly present on the external surface of the Co(II) oxide, obtainable according to one or more of claims 1 to 9.

12. Co(II) oxide containing metallic cobalt, wherein the metallic cobalt is present both on the external and on the internal surface of the Co(II) oxide, obtainable according to one or more of claims 1 to 9.

13. Use of the Co(II) oxide containing metallic cobalt according to claims 1 to 8 and 10 as an electrode material.

## Revendications

1. Procédé de production d'oxyde de Co(II) contenant du cobalt métallique, caractérisé en ce que des composés du cobalt basiques, cristallisés, de formule générale :
Co[ (OH)₂]ₐ[O]_{b}[CO₃]_{C}
où la somme a+b+c est ≥ 1 et ≤1,5,
sont mis à réagir avec des solutions aqueuses et/ou alcooliques d'un composé organique contenant au moins un radical carboxylique et les matières solides ainsi obtenues sont séparées de la suspension et calcinées.

2. Procédé suivant la revendication 1,
caractérisé en ce que l'on introduit comme composé organique contenant au moins un radical carboxylique, des acides carboxyliques.

3. Procédé suivant la revendication 2,
caractérisé en ce que l'on introduit comme acide carboxylique :
- des monoacides carboxyliques linéaires ou ramifiés, saturés ou insaturés, avec un nombre d'atomes C allant de 1 à 9, et/ou
- des polyacides carboxyliques linéaires ou ramifiés, saturés ou insaturés, avec un nombre d'atomes C allant de 2 à 10, et/ou
- des mono- et polyacides carboxyliques cycliques ou hétérocycliques, saturés ou insaturés, avec un nombre d'atomes C allant de 4 à 14, et/ou
- des mono- et polyacides hydroxycarboxyliques linéaires ou ramifiés, saturés ou insaturés, avec un nombre d'atomes C allant de 2 à 7, et/ou
- des acides hydroxycarboxyliques aromatiques, avec un nombre d'atomes C allant de 7 à 11, et/ou
- des acides cétocarboxyliques cycliques ou aliphatiques, saturés ou insaturés, avec un nombre d'atomes C allant de 2 à 14.

4. Procédé suivant l'une quelconque des revendications 2 ou 3, caractérisé en ce que l'on introduit comme acide carboxylique, de préférence, l'acide adipique, l'acide succinique, l'acide glutarique, l'acide glyoxylique, l'acide maléique, l'acide malonique, l'acide lactique, l'acide oxalique, l'acide phtalique, l'acide mucique, l'acide sorbique, l'acide tartrique racémique, l'acide versatique, l'acide tartrique droit et/ou l'acide citrique.

5. Procédé suivant l'une ou plusieurs des revendications 1 à 4, caractérisé en ce que l'on introduit comme composé organique contenant au moins un radical carboxylique, un acide carboxylique partiellement estérifié suivant les revendications 2 à 4.

6. Procédé suivant l'une ou plusieurs des revendications 1 à 5, caractérisé en ce que les composés du Co basiques, cristallisés présentent une granulométrie dans l'intervalle allant de 0,5 µm à 50 µm, de préférence de 2 à 20 µm.

7. Procédé suivant l'une ou plusieurs des revendications 1 à 6, caractérisé en ce que l'on introduit comme composés du Co basiques, cristallisés, ceux de morphologie sphérique.

8. Procédé suivant l'une ou plusieurs des revendications 1 à 7, caractérisé en ce que l'on réalise la calcination dans une atmosphère de gaz inerte à des températures situées dans l'intervalle allant de 200 à 1000°C, de préférence de 500 à 800°C.

9. Procédé suivant l'une ou plusieurs des revendications 1 à 8, caractérisé en ce que l'on effectue la réaction dans l'intervalle de température allant de 50 à 100°C, de préférence de 70 à 90°C, où lors de la calcination suivante, le cobalt métallique est formé de manière prédominante sur la surface externe de l'oxyde de Co(II).

10. Procédé suivant l'une ou plusieurs des revendications 1 à 8, caractérisé en ce que l'on effectue la réaction d'abord pendant 0,1 heure à 3 heures, de préférence pendant 0,5 à 1,5 heure, à température ambiante et ensuite, pendant 0,1 heure à 3 heures entre 50 et 100°C, de préférence entre 70 et 90°C, où après la calcination suivante, la surface non seulement interne, mais également externe de l'oxyde de Co(II) est revêtue de cobalt métallique.

11. Oxyde de Co(II) contenant du cobalt métallique, où le cobalt métallique est présent essentiellement sur la surface externe de l'oxyde de Co(II), pouvant être obtenu suivant l'une ou plusieurs des revendications 1 à 9.

12. Oxyde de Co(II) contenant du cobalt métallique, où le cobalt métallique est présent sur la surface non seulement externe, mais également interne de l'oxyde de Co(II), pouvant être obtenu suivant l'une ou plusieurs des revendications 1 à 9.

13. Utilisation de l'oxyde de Co(II) contenant du cobalt métallique suivant les revendications 1 à 8 et 10, comme matériau d'électrode dans les cellules secondaires.
